# EUROPEAN PATENT APPLICATION

(11) **EP 1 287 796 A1**
(43) Date of publication of application: **05.03.2003**
(21) Application number: 02292169.6
(22) Date of filing: 03.09.2002
(51) Int. Cl.: A61F 2/44

(54) **Intervertebral fusion device**

(30) Priority: 04.09.2001 CA 2356535
(71) Applicant: Biorthex Inc., Montreal, Quebec H4N 3H5 (CA)
(72) Inventor: Sicotte, Benoit, H7X 3H8 Laval, Quebec (CA); Leroux, Michel, H4B 2G2 Montreal, Quebec (CA); Quesnel, Sylvio, H8N 2L9 Lasalle, Quebec (CA)
(74) Representative: Texier, Christian

(57) **Abstract**

An intervertebral fusion device comprises a cylindrical body of porous TiNi and having a uni-directional porosity defined by a plurality of slender pores extending in a generally vertical direction across an intervertebral space between a pair of adjacent inferior and superior vertebrae.

## Description

### BACKGROUND OF THE INVENTION

### Field of the Invention

The present invention relates to medical implants of the type used in the fusion of two adjacent vertebrae and, more particularly, to such implants having osteoconductive properties.

### Description of the Prior Art

Low back pain often originate from degenerative disc diseases (DDD) or disorders in a spinal disc between two adjacent vertebrae. Different techniques are used for the surgical treatments of these types of conditions, one of which has been to fuse the two vertebrae surrounding the affected disc. In most cases, the disc is removed and a biocompatible structure is placed in the intradiscal space left by the removed spinal disc in order to stabilize the two surrounding vertebrae and prevent the intradiscal space from collapsing, which could results in damage to the nerves extending along the spinal column.

Over the years, numerous devices have been developed to fill an intervertebral space following removal of an intervertebral disc in order to prevent the disc space to collapse and to promote fusion of the adjacent vertebrae within the disc space.

It was first proposed to stabilize the spinal column with a metal plate or rod spanning the affected vertebrae and to fill the space left by the removed disc with allograft or autograft bone material in order to fuse the two adjacent vertebrae together.

Then, it was suggested to implant a fusion cage in the intradiscal space vacated by a removed disc in order to both stabilize and promote fusion of the vertebrae within the intradiscal space. This type of hollow implant is typically filled with autograft or allograft material prior to insertion into the intradiscal space and, thus, requires surgery to obtain the bone material.

Most recently, a porous tantalum implant allowing bone ingrowth without the aid of bonegraft has been developed. Such a porous implant is disclosed in United State Patent No. 5,282,861 issued to Kaplan on February 1, 1994.

Although the tantalum porous structure disclosed in the above mentioned patent constitutes a major advancement, it has been found that there is a need for a new porous implant having improved structural behavior when solicited both in tension and compression, and which favors fast osseointegration and long term survival of bone tissues within the implant.

### SUMMARY OF THE INVENTION

It is therefore an aim of the present invention to provide a porous implant having specific physical properties for promoting bone ingrowth.

It is also an aim of the present invention to provide a porous implant adapted to favor long-term survival of the bone tissues within the implant.

It is a further aim of the present invention to provide a porous implant adapted to promote bone tissue ingrowth into the implant without the aid of bonegrafts.

It is a still further aim of the present invention to provide a porous implant adapted to favor fast osseointegration.

Therefore, in accordance with the present invention, an intervertebral fusion device for implantation in an intradiscal space between adjacent superior and inferior vertebrae, comprising a body sized to be tightly fitted between adjacent superior and inferior vertebrae and having sufficient structural rigidity to maintain the vertebrae at a desired intradiscal space, said body being made of a porous biocompatible material adapted to promote bone tissue ingrowth into the body, wherein said body is characterized by a uni-directional pore distribution generally oriented to extend across the intradiscal space once the device has been positioned therein.

In accordance with a further general aspect of the present invention, there is provided an intervertebral fusion device comprising a body of porous TiNi and having a uni-directional porosity defined by a plurality of slender pores extending in an upstanding direction across an intervertebral space between a pair of adjacent inferior and superior vertebrae.

### BRIEF DESCRIPTION OF THE DRAWINGS

Having thus generally described the nature of the invention, reference will now be made to the accompanying drawings, showing by way of illustration a preferred embodiment thereof, and in which:
Fig. 1 is a schematic rear end view of a porous intervertebral fusion implant according to an embodiment of the present invention;
Fig. 2 is a schematic elevation view of the intervertebral fusion implant of Fig. 1;
Fig. 3 is a schematic posterior view of the spine showing two intervertebral fusion implants implanted within the intradiscal space between vertebrae L4 and L5; and
Fig. 4 is a schematic saggital plane view of one of the intervertebral fusion implant implanted between vertebrae L4 and L5 shown in Fig. 3.

### DESCRIPTION OF THE PREFERRED EMBODIMENTS

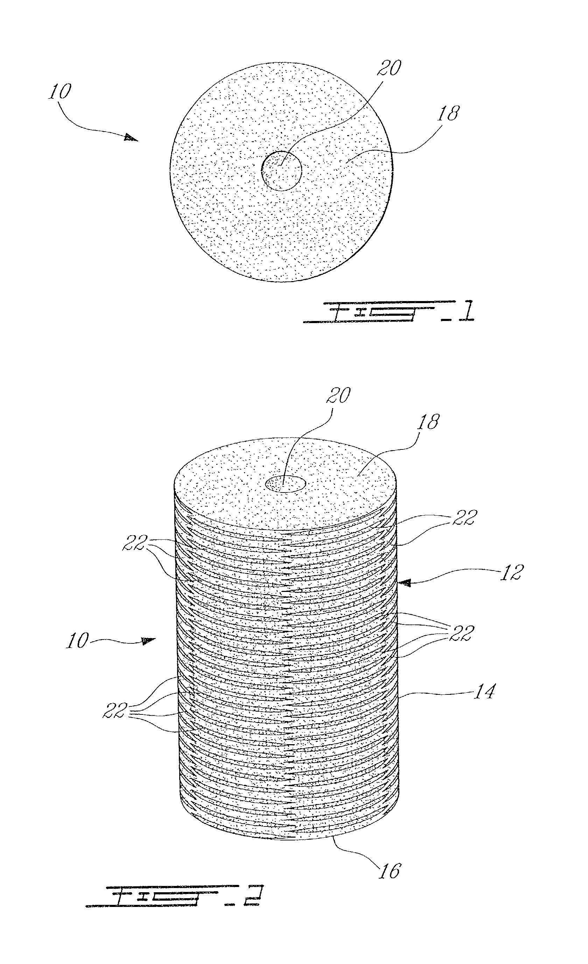

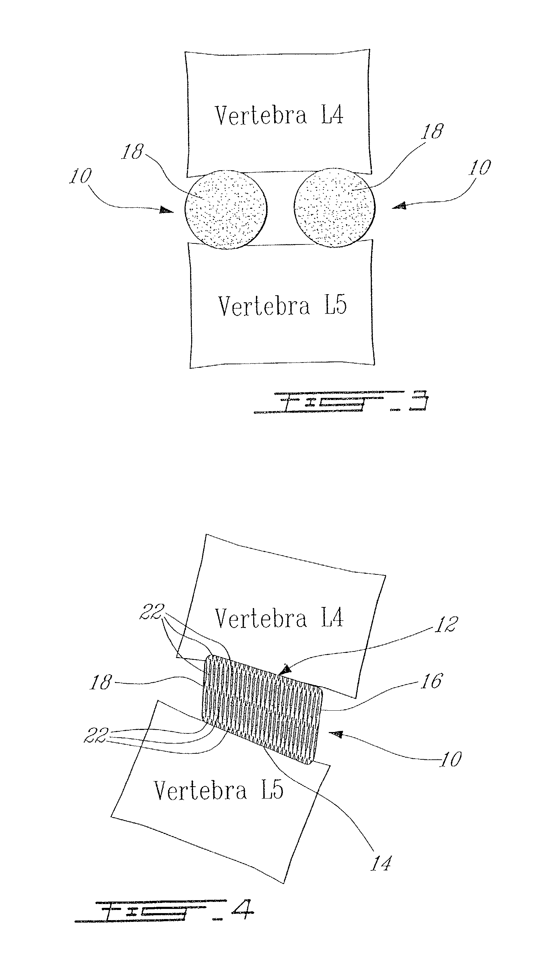

Figs. 1 and 2 illustrate a bone fusion implant 10 adapted to be inserted into the intervertebral space left after the removal of a damaged spinal disc in order to fuse the surrounding superior and inferior vertebrae.

The bone fusion implant 10 comprises a cylindrical body 12 having a side wall 14 extending between opposed anterior and posterior ends 16 and 18. A small central hole 20 is defined in the posterior end 18 of the cylindrical body 12 for engagement with a tool (not shown) that can be used to facilitate the positioning of the cylindrical body 12 between a pair of adjacent superior and inferior vertebrae.

The cylindrical body 12 is made of a biocompatible, porous, high strength material to permit bone ingrowth within the body 12 without the aid of bonegraft. In one preferred embodiment, the cylindrical body 12 is made of a suitable porous TiNi alloy (porous titanium-nickel alloy) of the type described in United States Patent No. 5,986,169 issued on November 16, 1999 to Gjunter, the teachings of which are incorporated herein by reference.

The porous TiNi alloy device has a porosity of 8 to 90% and preferably between about 60% and 70%. More particularly, as shown in Figs. 2 and 3, the porous body 12 has a uni-directional porosity (anisotropic porosity) defined by a plurality of passageways or elongated pores 22 extending throughout the body 12 in a direction substantially perpendicular to the longitudinal axis thereof so as to be substantially vertical once the implant 10 has been horizontally inserted between a pair of adjacent vertebrae (see Fig. 4). In this way, the porosity of the body 12 is generally oriented along the implant compression axis, which also corresponds to the direction of the bone tissue progression into the implant, i.e. across the intradiscal space. The orientation of the pores 22 does not have to be exactly perpendicular to the longitudinal axis of the body 12. Indeed, a difference of ±15 degrees is acceptable.
Since the implant 10 is horizontally inserted in position between the vertebrae, the resulting vertical orientation of the pores 22 also corresponds to a verticalization of the sidewalls of the pores 22, thereby enhancing the compressive strength of the implant 10. The improved compressive strength contributes to the longevity of the implant 10 and to the integrity of the intradiscal space.

The vertical orientation of the pores 22 also enhances the osteoconductive properties of the implant 10, thereby promoting faster osseointegration. This provides for a higher rate of bone ingrowth into the implant 10, thereby leading to faster skeleton fixation of the implant 10 and fusion of the vertebrae. In this way, dislodgement of the implant 10 should be prevented and the intervertebral space maintained. The stability of the implant and the absence of movement will promote the fusion of the posterior structures of the vertebrae and a reduction of the pain for the patient.

As can be appreciated from Figs. 2 and 4, the pores 22 are slender, i.e. they are small in cross section as compared with length. In addition of providing sufficient space for a complete integration of adjacent biological elements, this offers in a lengthwise direction of the implant 10, an optimal distance for allowing the adherence and growth of osseous cells on the body 12, thereby providing for a complete growth of the bone tissues across the implant 10. The shape of the pores 22 has a direct impact on the osteoconductive properties of the porous implant 10. The slender shape of the pores 22 provides for an improved interface between the bone and the implant 10, which leads to complete, strong and long-term osseointegration.

Porous TiNi is preferred to other porous biocompatible material, since it has a modulus of elasticity similar to that of cancellous bones, thereby providing for the dynamic stimulation of the bone tissues within the implant 10. This stimulation ensures a reaction of the bone tissues, which favors long-term survival of the bone tissues within the implant 10.

The porous TiNi may be produced in accordance with the procedures described in WO 01/13969 A1, published on March 1, 2001, the teachings of which are incorporated herein by reference. In summary, porous TiNi can be produced by using a hot rotational synthesis method including the steps of: **a)** drying raw powders of titanium and nickel under a vacuum state to remove moisture and surface absorption materials, **b)** dry-mixing the raw powders obtained from step a) with each other at a ratio of about 1:1 in atomic weight to manufacture mixed powders having uniform compositions, **c)** molding the mixed powders within a cylindrical quartz tube by compressing the mixed powders therein in accordance with the desired porosity and pore size, d) reacting the mixed powders obtained from step c) in a reaction furnace by a hot rotational synthesis method, **e)** cooling titanium-nickel products reacted in step d) using a reservoir for a cooling liquid, and **f)** removing impurities on a surface of the cooled titanium-nickel products and machining the products in a desired shape.

A porous cylindrical structure having the desired characteristics, namely a porosity generally perpendicular to the longitudinal axis of the cylindrical structure, slender pores and a modulus of elasticity similar to that of a cancellous bone in compression as well as in tension, may be obtained by: a) maintaining the ignition temperature at the initiation of the thermal reaction in a range of about 340 to about 400 degrees Celsius, and b) molding the mixed powders by first performing a partial compacting of the powders via a tapping technique followed by a final compacting of the powders through the use of a mechanical press. The powders are first compacted by tapping to a density of about 1.50 to about 1.60 gr/cm³ and then to a density of about 2.12 gr/cm³ ± 0.5 gr/cm³ through the use of the mechanical press.

In use, a set of implants 10 are inserted in compression in the intradiscal space left by a removed spinal disc between a pair of adjacent vertebrae, such as vertebrae L4 and L5, as shown in Fig. 3. The roughness of the sidewalls of the implants 10 is such that no external anchoring structure is needed to secure the implants 10 in position. Bone ingrowth into the slender pores 22 provides skeleton fixation for the permanent implants 10.

## Claims

1. An intervertebral fusion device for implantation in an intradiscal space between adjacent superior and inferior vertebrae, comprising a body sized to be tightly fitted between adjacent superior and inferior vertebrae and having sufficient structural rigidity to maintain the vertebrae at a desired intradiscal space, said body being made of a porous biocompatible material adapted to promote bone tissue ingrowth into the body, wherein said body is **characterized by** a uni-directional pore distribution generally oriented to extend across the intradiscal space once the device has been implanted therein.

2. An intervertebral fusion device as defined in claim 1, wherein said uni-directional pore distribution comprises a plurality of pores, said pores being slender.

3. An intervertebral fusion device as defined in claim 1, wherein said body is made of porous TiNi.

4. An intervertebral fusion device as defined in claim 1, wherein said uni-directional pore distribution is normal ± 15 degrees to a posterior/anterior axis of the implant.

5. An intervertebral fusion device as defined in claim 1, wherein said body is of cylindrical shape, and wherein said uni-directional pore distribution is transversal to a longitudinal axis of said body.

6. An intervertebral fusion device as defined in claim 5, wherein said uni-directional pore distribution is perpendicular ± 15 degrees to said longitudinal axis.

7. An intervertebral fusion device comprising a body of porous TiNi and having a uni-directional porosity defined by a plurality of slender pores extending in an upstanding direction across an intervertebral space between a pair of adjacent inferior and superior vertebrae.
